# EUROPÄISCHE PATENTANMELDUNG

(11) **EP 4 494 615 A1**
(43) Veröffentlichungstag der Anmeldung: **22.01.2025**
(21) Anmeldenummer: 23186438.0
(22) Anmeldetag: 19.07.2023
(51) Int. Cl.: A61F 9/02, A61F 9/06

(54) **LASERSCHUTZVORRICHTUNG, INSBESONDERE LASERSCHUTZHELM**

(71) Anmelder: Optrel Holding AG, 9630 Wattwil (CH)
(72) Erfinder: WIRZ, Simon, 9052 Niederteufel (CH); ESPOSITO, Martin, 8640 Rapperswil (CH); HEUSSER, Jonathan, 8713 Uerikon (CH)
(74) Vertreter: Daub, Thomas

(57) **Zusammenfassung**

Die Erfindung geht aus von einer Laserschutzvorrichtung, insbesondere Laserschutzhelm, mit zumindest einem optischen Blendschutzfilter (12), welcher insbesondere einen Nasenausschnitt (14) aufweist, mit zumindest einer Sensoreinheit (16), welche zu einer Erfassung eines Arbeitszustands und einer davon abhängigen Ansteuerung einer Durchlässigkeit des Blendschutzfilters (12) vorgesehen ist, mit zumindest einer Schildeinheit (18), welche dazu vorgesehen ist, ein Gesicht eines Benutzers (20) zu schützen und in welcher der zumindest eine Blendschutzfilter (12) fest aufgenommen ist.

Es wird vorgeschlagen, dass die Laserschutzvorrichtung eine Strahlenschutzeinheit (22) aufweist, welche sich um den Blendschutzfilter (12) erstreckt und zumindest teilweise aus einem metallischen Werkstoff besteht.

## Beschreibung

### Stand der Technik

Die Erfindung betrifft eine Laserschutzvorrichtung, insbesondere einen Laserschutzhelm.

Es ist bereits eine Laserschutzvorrichtung, insbesondere ein Laserschutzhelm, mit zumindest einem optischen Blendschutzfilter, welcher insbesondere einen Nasenausschnitt aufweist, mit zumindest einer Sensoreinheit, welche zu einer Erfassung eines Arbeitszustands und einer davon abhängigen Ansteuerung einer Durchlässigkeit des Blendschutzfilters vorgesehen ist, mit zumindest einer Schildeinheit, welche dazu vorgesehen ist, ein Gesicht eines Bedieners zu schützen und in welcher der zumindest eine Blendschutzfilter fest aufgenommen ist, vorgeschlagen worden.

Die Aufgabe der Erfindung besteht insbesondere darin, eine gattungsgemäße Vorrichtung mit verbesserten Eigenschaften hinsichtlich eines Schutzes und/oder eines Komforts bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Laserschutzvorrichtung, insbesondere Laserschutzhelm, mit zumindest einem optischen Blendschutzfilter, welcher insbesondere einen Nasenausschnitt aufweist, mit zumindest einer Sensoreinheit, welche zu einer Erfassung eines Arbeitszustands und einer davon abhängigen Ansteuerung einer Durchlässigkeit des Blendschutzfilters vorgesehen ist, mit zumindest einer Schildeinheit, welche dazu vorgesehen ist, ein Gesicht eines Bedieners zu schützen und in welcher der zumindest eine Blendschutzfilter fest aufgenommen ist.

Es wird vorgeschlagen, dass die Laserschutzvorrichtung eine Strahlenschutzeinheit, welche sich um den Blendschutzfilter erstreckt und zumindest teilweise aus einem laserabsorbierenden, insbesondere metallischen, Werkstoff besteht, aufweist.

Unter einer "Laserschutzvorrichtung" soll in diesem Zusammenhang insbesondere eine Vorrichtung verstanden werden, welche zu einem Schutz eines Benutzers vor zu großer Helligkeit und/oder Funken und/oder einem Laserstrahl, insbesondere Laserschweißstrahl, vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses, insbesondere eines Laserschweißprozesses, dient. Es sind verschiedene, einem Fachmann als sinnvoll erscheinende Ausgestaltungen einer Laserschutzvorrichtung denkbar, wie beispielsweise als Laserschweißhelm, -schirm, - maske und/oder -schild. Ferner soll in diesem Zusammenhang unter einem "optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter, insbesondere Laserschweißschutzfilter, mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einem "Nasenausschnitt" in diesem Zusammenhang insbesondere eine immaterielle Aussparung in einem zumindest teilweise transluzenten Teilbereich des Blendschutzfilters verstanden werden, welcher in zumindest einer Betriebsstellung der Laserschutzvorrichtung zumindest teilweise zu einer zumindest teilweisen Aufnahme einer Nase eines Benutzers vorgesehen ist. Vorzugsweise ist die Aussparung in jedem Punkt in zumindest einer Ebene, insbesondere parallel zu einer Haupterstreckungsebene des Blendschutzfilters, in einem Winkelbereich von zumindest 180° von einem materiellen Teilbereich, insbesondere einem zumindest teilweise transluzenten Teilbereich des Blendschutzfilters umgeben. Vorzugsweise umgreift der Blendschutzfilter in zumindest einer Betriebsstellung der Laserschutzvorrichtung eine Nase des Benutzers. Besonders bevorzugt beträgt eine vertikale Erstreckung des Nasenausschnitts zumindest 10%, vorzugsweise zumindest 30%, bevorzugt zumindest 50% und besonders bevorzugt zumindest 55% einer vertikalen Erstreckung des Blendschutzfilters. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoreinheiten denkbar. Vorzugsweise weist die Sensoreinheit insbesondere zumindest eine Fotozelle auf. Bevorzugt ist die Fotozelle insbesondere zumindest zu einer optischen Detektion eines Lichtbogens vorgesehen. Ferner soll in diesem Zusammenhang unter einer "Schildeinheit" insbesondere eine Einheit verstanden werden, welche in einer regulären Betriebsstellung vor einem Gesicht des Benutzers angeordnet ist. Vorzugsweise soll darunter insbesondere eine Einheit verstanden werden, welche in einer Betriebsstellung der Laserschutzvorrichtung insbesondere zumindest einen wesentlichen Teil eines Gesichts eines Benutzers verdeckt. Vorzugsweise soll darunter insbesondere eine Einheit verstanden werden, welche zu einem Schutz eines Gesichts, wie beispielsweise vor umherfliegenden Funken, vorgesehen ist. Bevorzugt ist die Schildeinheit insbesondere dazu vorgesehen, eine Schutzbarriere zwischen einem Arbeitsbereich und einem Gesicht des Benutzers zu bilden. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Unter einer "Strahlenschutzeinheit" soll in diesem Zusammenhang insbesondere eine Einheit verstanden werden, welche zu einem Schutz der Augen eines Benutzers vor Strahlung, insbesondere Laserstrahlung, vorgesehen ist, welche insbesondere an dem optischen Blendschutzfilter vorbei auf zumindest ein Auge des Benutzers gerichtet ist. Vorzugsweise ist die Strahlenschutzeinheit dazu vorgesehen, ein peripheres Sichtfeld und/oder ein Gesicht eines Benutzers vor Laserstrahlung zu schützen. Die Strahlenschutzeinheit ist insbesondere dazu vorgesehen, die Augen des Benutzers in einem Bereich um den optischen Blendschutzfilter abzuschirmen. Die Strahlenschutzeinheit ist insbesondere für Laserstrahlung undurchdringlich ausgebildet. Insbesondere kann die Laserstrahlung dagegen die Schildeinheit durchdringen. Unter einem "laserabsorbierenden Material" soll in diesem Zusammenhang insbesondere ein Material verstanden welches Laserstrahlung zumindest in einem definierten Wellenlängenbereich, insbesondere vollständig, absorbiert und/oder reflektiert. Dabei sind verschiedene, einem Fachmann als sinnvoll erscheinende Materialien denkbar, wie beispielsweise metallische Werkstoffe, insbesondere Aluminium und/oder Aluminiumlegierungen. Vorzugsweise ist das laserabsorbierende Material für Laserstrahlung zumindest im Wesentlichen, insbesondere vollständig, undurchlässig.

Durch die erfindungsgemäße Laserschutzvorrichtung kann insbesondere ein vorteilhafter Arbeiterschutz während des Laserprozesses, insbesondere eines Laserschweißprozesses ermöglicht werden. Es kann insbesondere eine Schädigung der Augen eines Benutzers durch einen falsch gerichteten Laserstrahl vermieden werden. Gleichzeitig kann insbesondere auch ein mechanischer Schutz eines Gesichts des Benutzers, beispielsweise vor umherfliegenden Teilen oder Funken, ermöglicht werden. Ferner kann ein vorteilhaft hoher Bedien- und/oder Tragekomfort ermöglicht werden.

Ferner wird vorgeschlagen, dass die Strahlenschutzeinheit zumindest eine sich um den Blendschutzfilter erstreckende Strahlenschutzwandung aufweist, welche aus einem laserabsorbierenden, insbesondere metallischen, Werkstoff besteht. Die Strahlenschutzwandung ist insbesondere von einem geformten Blechteil und/oder einem Gussteil gebildet, welches sich an einer Außenkante des optischen Blendschutzfilters um den optischen Blendschutzfilter erstreckt. Bevorzugt liegt die Strahlenschutzwandung direkt an den Blendschutzfilter an. Die Strahlenschutzwandung kann insbesondere vollständig aus Metall oder beispielsweise aus einem Verbundmaterial bestehen. Vorzugsweise ist die Strahlenschutzwandung insbesondere von einem schmalen, umlaufenden Steg gebildet, welcher in einem getragenen Zustand dazu vorgesehen ist, sich in Richtung eines Gesichts des Benutzers zu erstrecken. Dadurch kann insbesondere eine vorteilhaft sichere und leichte Strahlenschutzeinheit bereitgestellt werden.

Des Weiteren wird vorgeschlagen, dass die zumindest eine Strahlenschutzwandung aus Aluminium und/oder einer Aluminiumlegierung besteht. Vorzugsweise besteht die Strahlenschutzwandung vollständig aus Aluminium oder einer Aluminiumlegierung. Es sind jedoch auch andere, einem Fachmann als sinnvoll erscheinende Materialien denkbar. Dadurch kann insbesondere eine vorteilhaft sichere und leichte Strahlenschutzeinheit bereitgestellt werden.

Es wird ferner vorgeschlagen, dass die zumindest eine Strahlenschutzwandung zumindest im Wesentlichen senkrecht zu einer Haupterstreckungsebene des Blendschutzfilters in einen Innenraum der Schildeinheit ragt. Vorzugsweise ragt die zumindest eine Strahlenschutzwandung in einem getragenen Zustand zumindest im Wesentlichen senkrecht zu einer Haupterstreckungsebene des Blendschutzfilters in einen Innenraum der Schildeinheit in Richtung eines Gesichts des Benutzers. Der Ausdruck "im Wesentlichen senkrecht" soll hier insbesondere eine Ausrichtung einer Richtung relativ zu einer Bezugsrichtung definieren, wobei die Richtung und die Bezugsrichtung, insbesondere in einer Projektionsebene betrachtet, einen Winkel von 90° einschließen und der Winkel eine maximale Abweichung von insbesondere kleiner als 16°, vorteilhaft kleiner als 12° bevorzugt keiner als 8° und besonders vorteilhaft kleiner als 2° aufweist. Unter einer "Haupterstreckungsebene" einer Baueinheit soll insbesondere eine Ebene verstanden werden, welche parallel zu einer größten Seitenfläche eines kleinsten gedachten Quaders ist, welcher die Baueinheit gerade noch vollständig umschließt, und insbesondere durch den Mittelpunkt des Quaders verläuft. Die Strahlenschutzwandung bildet insbesondere einen Nasenausschnitt aus, in welchem die Strahlenschutzwandung eine verringerte Höhe aufweist. Neben dem Nasenausschnitt weist die Strahlenschutzwandung insbesondere eine zumindest annähernd konstante Höhe auf. Dadurch kann insbesondere eine vorteilhaft sichere Abschirmung bereitgestellt werden. Es kann insbesondere eine vorteilhaft sichere Strahlenschutzeinheit bereitgestellt werden. Ferner kann ein vorteilhaft hoher Tragekomfort bereitgestellt werden.

Es wird weiter vorgeschlagen, dass die zumindest eine Strahlenschutzeinheit zumindest einen Schildeinsatz aufweist, welcher aus einem laserabsorbierenden, insbesondere metallischen, Werkstoff besteht. Vorzugsweise besteht der Schildeinsatz zumindest zu einem Großteil aus Aluminium und/oder einer Aluminiumlegierung. Der Schildeinsatz ist insbesondere direkt mit der Schildeinheit gekoppelt und verstärkt diese. Vorzugsweise bildet der Schildeinsatz eine bereichsweise Zweiwandigkeit der Schildeinheit aus. Bevorzugt liegt der Schildeinsatz an einer Innenseite der Schildeinheit an der Schildeinheit an, wobei der Schildeinsatz insbesondere einer Form der Schildeinheit folgt. Ferner wird vorgeschlagen, dass der Schildeinsatz in einem Mundbereich eines Benutzers hinter der Schildeinheit angeordnet ist. Die Schildeinheit weist insbesondere einen Stirnbereich, einen Kinnbereich sowie einen zwischen dem Stirnbereich und dem Kinnbereich angeordneten Augenbereich auf. Der Stirnbereich, der Kinnbereich und der Augenbereich sind bevorzugt einstückig ausgebildet. Vorzugsweise sind der Stirnbereich und der Kinnbereich auf einer dem Gesicht des Benutzers abgewandten Außenseite im Wesentlichen konvex geformt. Der Schildeinsatz ist insbesondere in dem Kinnbereich der Schildeinheit angeordnet. Dadurch kann insbesondere eine vorteilhaft sichere Abschirmung bereitgestellt werden. Es kann insbesondere eine vorteilhaft sichere Strahlenschutzeinheit bereitgestellt werden. Ferner kann ein vorteilhaft hoher Tragekomfort bereitgestellt werden.

Es wird weiter vorgeschlagen, dass die Strahlenschutzeinheit von der Schildeinheit verschieden ist. Die Strahlenschutzeinheit ist insbesondere von einem separaten Bauteil gebildet. Die Strahlenschutzeinheit ist insbesondere mit der Schildeinheit verbunden. Die Strahlenschutzeinheit ist insbesondere über eine Schutzkassette mit der Schildeinheit verbunden. Die Schildeinheit besteht insbesondere aus einem von der Strahlenschutzeinheit verschiedenen Material. Die Schildeinheit besteht insbesondere zumindest teilweise aus Kunststoff. Vorzugsweise besteht die Schildeinheit vollständig aus Kunststoff. Die Strahlenschutzeinheit ist insbesondere lösbar mit der Schildeinheit verbunden. Dadurch kann insbesondere ein vorteilhaft modularer Aufbau der Laserschutzvorrichtung bereitgestellt werden. Es kann insbesondere ein vorteilhafter Schutz des Benutzers ermöglicht werden.

Ferner wird vorgeschlagen, dass die Laserschutzvorrichtung eine Kopfbefestigungseinheit zu einer Befestigung an einem Kopf eines Benutzers und eine Einstelleinheit aufweist, durch welche eine Position der zumindest einen Schildeinheit definiert relativ zu der zumindest einen Kopfbefestigungseinheit beweglich ausgeführt ist. In diesem Zusammenhang soll unter einer "Kopfbefestigungseinheit" insbesondere eine Einheit verstanden werden, welche zu einer Befestigung, insbesondere einer Fixierung der Laserschutzvorrichtung an einem Kopf des Benutzers vorgesehen ist. Vorzugsweise ist die Einheit hierzu dazu vorgesehen, einen Kopf des Benutzers zumindest teilweise zu umgreifen. In diesem Zusammenhang soll unter "zumindest teilweise umgreifen" insbesondere verstanden werden, dass ein Kopf in zumindest einer Ebene in einem Winkelbereich von zumindest 160°, vorzugsweise von zumindest 180°, bevorzugt von zumindest 270° und besonders bevorzugt von zumindest 360° von der Kopfbefestigungseinheit umschlossen wird. Besonders bevorzugt ist die Kopfbefestigungseinheit in einem regulären Betriebszustand dazu vorgesehen, zumindest an einer Stirn sowie einem Hinterkopf eines Benutzers anzuliegen. Ferner soll in diesem Zusammenhang unter einer "Einstelleinheit" insbesondere eine Einheit verstanden werden, mittels welcher insbesondere manuell eine Position der zumindest einen Schildeinheit definiert relativ zu der zumindest einen Kopfbefestigungseinheit einstellbar ausgeführt ist. Vorzugsweise weist die Einstelleinheit zumindest ein Einstellmittel auf, über welches ein Benutzer eine Position und/oder eine Lage der Schildeinheit definiert relativ zu der zumindest einen Kopfbefestigungseinheit verändern kann. Besonders bevorzugt ist die Einstelleinheit insbesondere dazu vorgesehen, eine Bewegung der zumindest einen Schildeinheit definiert relativ zu der zumindest einen Kopfbefestigungseinheit zumindest zu führen. Grundsätzlich ist auch denkbar, dass die Schildeinheit direkt relativ zu der Kopfbefestigungseinheit bewegt wird, wobei eine Bewegung dabei insbesondere durch die Einstelleinheit definiert geführt wird. Dabei soll unter "definiert" insbesondere verstanden werden, dass eine Bewegung der Schildeinheit geführt wird. Vorzugsweise soll darunter zudem verstanden werden, dass eine Grenze einer Bewegung der Schildeinheit relativ zu der zumindest einen Kopfbefestigungseinheit durch die Einstelleinheit vorgegeben ist. Besonders bevorzugt soll darunter insbesondere verstanden werden, dass die Schildeinheit, insbesondere innerhalb der Grenzen der Beweglichkeit, relativ zu der zumindest einen Kopfbefestigungseinheit in mehreren Positionen festlegbar, insbesondere arretierbar ist. Dadurch kann insbesondere eine vorteilhaft variable Einstellung einer Position der Schildeinheit erreicht werden. Insbesondere kann dadurch erreicht werden, dass die Schildeinheit bedienerspezifisch optimal positioniert werden kann. Hierdurch kann der optische Blendschutzfilter vorzugsweise vorteilhaft klein ausgeführt und/oder ein Sichtfeld vorteilhaft groß ausgeführt werden.

Des Weiteren wird vorgeschlagen, dass mittels der zumindest einen Einstelleinheit eine direkte Distanz zwischen den Augen des Benutzers und dem optischen Blendschutzfilter einstellbar ausgebildet ist. Vorzugsweise ist mittels der zumindest einen Einstelleinheit eine Position der zumindest einen Schildeinheit in zumindest zwei Betriebsstellungen relativ zu der zumindest einen Kopfbefestigungseinheit fixierbar ausgeführt. Unter einer "Betriebsstellung" soll in diesem Zusammenhang insbesondere eine Stellung der Schildeinheit verstanden werden, in welcher die Schildeinheit eine vorgesehene Schutzfunktion ausführt und/oder ausführen kann. Vorzugsweise soll darunter insbesondere eine Stellung verstanden werden, in welcher die Schildeinheit vor einem Gesicht eines Benutzers angeordnet ist. Besonders bevorzugt unterscheiden sich die zumindest zwei fixierbaren Betriebsstellungen insbesondere durch eine Entfernung zwischen dem optischen Blendschutzfilter und den Augen des Benutzers und/oder in einem Winkel zwischen der Schildeinheit und der Kopfbefestigungseinheit und/oder in einer Höhe, in welcher der optische Blendschutzfilter relativ zu dem Kopf des Benutzers angeordnet ist. Vorzugsweise ist mittels der zumindest einen Einstelleinheit eine Distanz zwischen den Augen des Benutzers und dem optischen Blendschutzfilter in einem Betrieb senkrecht zu einer Kopfachse einstellbar. Bevorzugt ist mittels der zumindest einen Einstelleinheit eine Distanz zwischen den Augen des Benutzers und dem optischen Blendschutzfilter in einem Betrieb annähernd senkrecht zu einer Haupterstreckungsebene des Blendschutzfilters einstellbar. Unter einer "direkten Distanz" soll in diesem Zusammenhang insbesondere eine Distanz zwischen den Augen des Benutzers und dem optischen Blendschutzfilter während eines Betriebs, daher bei direktem Blickkontakt, verstanden werden. Dadurch kann insbesondere eine vorteilhaft variable Einstellung einer Position der Schildeinheit erreicht werden. Vorzugsweise kann dadurch erreicht werden, dass die Schildeinheit bedienerspezifisch optimal positioniert werden kann. Je nach Kopfumfang und Wohlbefinden des Schweißers kann dadurch vorteilhaft die Längsposition des optischen Blendschutzfilters eingestellt werden. Ferner kann dadurch eine Längsposition des optischen Blendschutzfilters auch für Brillenträger optimal angepasst werden. Umso näher der optische Blendschutzfilter am Gesicht angeordnet wird, desto größer wird das Sichtfeld für den Benutzer.

Es wird weiter vorgeschlagen, dass die zumindest eine Strahlenschutzeinheit zumindest einen Gesichtsanschlag aufweist, welcher in einer Endposition der Strahlenschutzeinheit dazu vorgesehen ist, ein Gesicht des Benutzers zu kontaktieren. Unter einem "Gesichtsanschlag" soll in diesem Zusammenhang insbesondere ein Anschlag verstanden werden, welcher dazu vorgesehen ist, zu einer Definition der Endposition der Strahlenschutzeinheit relativ zu einem Gesicht des Benutzers, ein Gesicht des Benutzers zu kontaktieren. Vorzugsweise soll darunter insbesondere ein Anschlag verstanden werden, dessen Endposition abhängig von einer Form eines Gesichts des Benutzers ist. Besonders bevorzugt soll darunter insbesondere ein Anschlag verstanden werden, bei welchem eine Anschlagposition durch eine Berührung zwischen Gesicht und Gesichtsanschlag definiert ist. Hierdurch kann zuverlässig eine Endposition der Strahlenschutzeinheit definiert werden. Ferner kann dadurch zuverlässig die Strahlenschutzeinheit in eine Betriebsstellung gebracht werden. Insbesondere kann dadurch eine optimale Positionierung der Strahlenschutzeinheit relativ zu einem Gesicht des Benutzers erreicht werden. Hierdurch kann wiederum eine optimale Abschirmung erreicht werden. Es kann vorzugsweise ein gesichtsspezifischer Anschlag bereitgestellt werden. Vorzugsweise ist der zumindest eine Gesichtsanschlag von einer Nasenauflage gebildet, welche in zumindest einer Betriebsstellung zu einer Kontaktierung einer Nase eines Benutzers vorgesehen ist. Vorzugsweise ist die Nasenauflage in zumindest einer Betriebsstellung zu einer Kontaktierung eines Nasenrückens eines Benutzers vorgesehen. Bevorzugt ist die Nasenauflage in zumindest einer Betriebsstellung zu einer Kontaktierung einer Nasenpyramide einer Nase eines Benutzers vorgesehen. Besonders bevorzugt ist die Nasenauflage von einer brillenähnlichen Nasenauflage gebildet. Vorzugsweise weist die Nasenauflage zumindest ein Nasenpad auf. Bevorzugt weist die Nasenauflage zumindest zwei Nasenpads auf. Besonders bevorzugt sind/ist eine Position und/oder eine Lage der Nasenpads einstellbar. Die Nasenauflage ist insbesondere an eine Form einer Nase anpassbar. Hierdurch kann zuverlässig eine Endposition der Schildeinheit definiert werden. Insbesondere kann dadurch eine optimale Positionierung der Strahlenschutzeinheit relativ zu einem Gesicht des Benutzers erreicht werden. Vorzugsweise kann eine Position der Schildeinheit an einer Gesichtsmitte ausgerichtet werden. Es kann eine zuverlässige Positionierung der Strahlenschutzeinheit relativ zu den Augen des Benutzers erreicht werden. Zudem kann zumindest ein geringer Teil eines Gewichts der Laserschutzvorrichtung auf der Nase abgestützt werden. Hierdurch kann insbesondere ein Nach-unten-Rutschen der Laserschutzvorrichtung während eines Betriebs vermieden werden.

Ferner geht die Erfindung aus von der Strahlenschutzeinheit der Laserschutzvorrichtung. Die Strahlenschutzeinheit kann insbesondere austauschbar mit der Schildeinheit der Laserschutzvorrichtung verbunden sein.

Die erfindungsgemäße Laserschutzvorrichtung soll hierbei nicht auf die oben beschriebene Anwendung und Ausführungsform beschränkt sein. Insbesondere kann die erfindungsgemäße Laserschutzvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: die erfindungsgemäße Laserschutzvorrichtung mit einer Schildeinheit, mit einer Kopfbefestigungseinheit, mit einem optischen Blendschutzfilter und mit einer Vorsatzscheibe auf einem Kopf eines Benutzers in einer schematischen Darstellung,
- Fig. 2: die erfindungsgemäße Laserschutzvorrichtung mit der Schildeinheit, mit der Kopfbefestigungseinheit, mit dem optischen Blendschutzfilter und mit einer Strahlenschutzeinheit in einer schematischen Rückansicht,
- Fig. 3: die Strahlenschutzeinheit der erfindungsgemäßen Laserschutzvorrichtung in einer schematischen Darstellung,
- Fig. 4: die Strahlenschutzeinheit der erfindungsgemäßen Laserschutzvorrichtung in einer weiteren schematischen Darstellung und
- Fig. 5: eine schematische Schnittansicht der Laserschutzvorrichtung mit dem optischen Blendschutzfilter im getragenen Zustand.

### Beschreibung des Ausführungsbeispiels

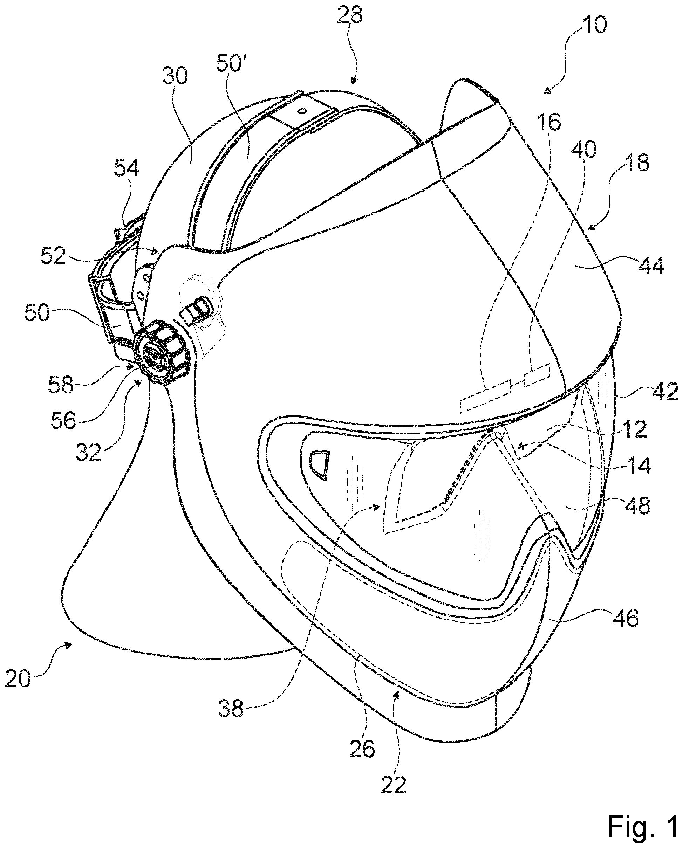

Die Figur 1 zeigt eine Laserschutzvorrichtung 10 auf einem Kopf 30 eines Benutzers 20. Die Laserschutzvorrichtung 10 ist dazu vorgesehen, während eines Betriebs von einem Benutzer 20 auf dem Kopf 30 getragen zu werden. Figur 1 zeigt die Laserschutzvorrichtung 10 in einer Betriebsstellung. Die Laserschutzvorrichtung 10 ist von einem Laserschweißhelm gebildet. Grundsätzlich wäre jedoch auch eine andere, einem Fachmann als sinnvoll erscheinende Ausbildung der Laserschutzvorrichtung 10 denkbar.

Die Laserschutzvorrichtung 10 weist einen optischen Blendschutzfilter 12 auf. Ferner weist die Laserschutzvorrichtung 10 eine Sensoreinheit 16 auf. Die Sensoreinheit 16 ist zu einer Erfassung eines Arbeitszustands und einer davon abhängigen Ansteuerung einer Durchlässigkeit des Blendschutzfilters 12 vorgesehen. Die Sensoreinheit 16 weist zumindest einen Sensor auf, der dazu vorgesehen ist, einen Schweißvorgang oder das Auftreten eines hellen Lichts, welches Augen 34 eines Benutzers 20 schädigen oder auf andere Weise beeinflussen könnte, zu detektieren. Der Sensor der Sensoreinheit 16 ist von einer Fotozelle gebildet. Grundsätzlich wäre jedoch auch einen andere, einem Fachmann als sinnvoll erscheinende Ausbildung des Sensors der Sensoreinheit 16 denkbar. Des Weiteren weist die Laserschutzvorrichtung 10 eine Schutzkassette 38 auf. Der optische Blendschutzfilter 12 und die Sensoreinheit 16 bilden einen Teil der Schutzkassette 38. Ferner weist die Schutzkassette 38 eine Elektronikeinheit 40 auf. Die Elektronikeinheit 40 ist mit der Sensoreinheit 16 gekoppelt. Die Elektronikeinheit 40 ist dazu vorgesehen, die Daten der Sensoreinheit 16 zu verarbeiten und abhängig davon den Blendschutzfilter 12 anzusteuern. Die Elektronikeinheit 40 umfasst zu einer Energieversorgung eine nicht weiter sichtbare Batterie und/oder Solarzelle.

Der optische Blendschutzfilter 12 ist von einem elektrooptischen Filter gebildet. Der optische Blendschutzfilter 12 ist von einem automatic darkening filter, kurz ADF, gebildet. Der optische Blendschutzfilter 12 besteht aus mehreren Schichten. Der optische Blendschutzfilter 12 ist als ein Mehrschichtverbund ausgebildet. Eine Anzahl von Schichten ist hierbei lediglich beispielhaft und kann grundsätzlich variieren.

Der Blendschutzfilter 12 weist eine rechteckige Grundform auf. Eine vertikale Erstreckung des Blendschutzfilters 12 ist dabei, in einem regulären Betriebszustand, geringer, als eine horizontale Erstreckung des Blendschutzfilters 12. Ferner weist der Blendschutzfilter 12 einen Nasenausschnitt 14 auf. Der Nasenausschnitt 14 ist von einer immateriellen Aussparung in einem materiellen Teil des Blendschutzfilters 12 gebildet. Der Nasenausschnitt 14 ist von einer immateriellen Aussparung in einem materiellen, zumindest teilweise transluzenten Teilbereich des Blendschutzfilters 12 gebildet. Der Nasenausschnitt 14 ist in einer Betriebsstellung der Laserschutzvorrichtung 10 zu einer teilweisen Aufnahme einer Nase des Benutzers 20 vorgesehen. Der Blendschutzfilter 12 umgreift in einer Betriebsstellung im Bereich des Nasenausschnitts 14 teilweise die Nase des Benutzers 20. Der Blendschutzfilter 12 weist eine im Wesentlichen rechteckige Grundform auf, wobei der Nasenausschnitt 14 in die rechteckige Grundform ragt. Der Nasenausschnitt 14 weist eine im Wesentlichen dreieckige Form auf. Die beiden durch den materiellen Teil des Blendschutzfilters 12 begrenzten Seiten weisen einen Winkel von annähernd 54° zueinander auf.

Ferner weist die Laserschutzvorrichtung 10 eine Schildeinheit 18 auf. Der Blendschutzfilter 12 ist fest in der Schildeinheit 18 aufgenommen. Der Blendschutzfilter 12 ist positionsfest in der Schildeinheit 18 aufgenommen. Der Blendschutzfilter 12 ist in einer Ausnehmung in der Schildeinheit 18 eingepasst. Die gesamte Schutzkassette 38 ist in einer Ausnehmung in der Schildeinheit 18 eingepasst. Die Schildeinheit 18 besteht aus einem im Wesentlichen formfesten Material. Die Schildeinheit 18 besteht aus einem Kunststoff, welcher insbesondere beständig gegenüber Funken und/oder anderen beim Schweißen auftretenden Einflüssen ist. Die Schildeinheit 18 ist dazu vorgesehen, insbesondere nach den einschlägigen Normen für Schweißmasken, ein Gesicht und/oder den Kopf 30 des Benutzers 20 zu bedecken und zu schützen. Die Schildeinheit 18 weist eine teilweise einer Kopfform angepasste Form auf. Die Schildeinheit 18 ist in einem getragenen Zustand der Laserschutzvorrichtung 10 teilweise um ein Gesicht des Benutzers 20 gebogen. Die Schildeinheit 18 nimmt in einem getragenen Zustand der Laserschutzvorrichtung 10 teilweise ein Gesicht des Benutzers 20 auf. Die Schildeinheit 18 weist einen Stirnbereich 44, einen Kinnbereich 46 sowie einen zwischen dem Stirnbereich 44 und dem Kinnbereich 46 angeordneten Augenbereich 48 auf. Der Stirnbereich 44, der Kinnbereich 46 und der Augenbereich 48 sind einstückig ausgebildet. Der Stirnbereich 44 und der Kinnbereich 46 sind auf einer dem Gesicht des Benutzers 20 abgewandten Außenseite im Wesentlichen konvex geformt. Der Augenbereich 48 weist eine Aufnahme zur Aufnahme der Schutzkassette 38 auf. Der Augenbereich 48 ist zu der Aufnahme der Schutzkassette 38 hin vertieft. Die Aufnahme für die Schutzkassette 38 ist gegenüber einer Grundform der Schildeinheit 18 nach innen, daher zu einem Gesicht des Benutzers 20 hin, versetzt angeordnet. Die Aufnahme für die Schutzkassette 38 ist gegenüber dem Stirnbereich 44 sowie gegenüber dem Kinnbereich 46 nach innen versetzt angeordnet.

Die Laserschutzvorrichtung 10 weist ferner eine Vorsatzscheibe 42 auf. Die Vorsatzscheibe 42 ist mit der Schildeinheit 18 über nicht weiter sichtbare Rastelemente verbunden. Vorzugsweise weist die Vorsatzscheibe 42 zwei gegenüberliegende Rastausnehmungen auf, in welche jeweils ein Rastelement der Schildeinheit 18 rastend eingreift. Durch die Verrastung kann die Vorsatzscheibe 42 einfach demontiert werden. Hierdurch sind eine einfache Reinigung und/oder ein einfacher Austausch möglich. Die Vorsatzscheibe 42 ist transparent ausgebildet. Die Vorsatzscheibe 42 ist zu einem Schutz der Schutzkassette 38 vorgesehen. Die Vorsatzscheibe 42 verdeckt die Schutzkassette 38 von außen. Hierdurch kann eine Beschädigung der Schutzkassette 38 vermieden werden. Bei einer Beschädigung der Vorsatzscheibe 42 kann diese, insbesondere im Gegensatz zu der Schutzkassette 38, einfach und kostengünstig ausgetauscht werden.

Des Weiteren weist die Laserschutzvorrichtung 10 eine Kopfbefestigungseinheit 28 auf. Die Kopfbefestigungseinheit 28 ist zu einer Befestigung an dem Kopf 30 des Benutzers 20 vorgesehen. Die Kopfbefestigungseinheit 28 ist von einem Kopfband gebildet. Die Kopfbefestigungseinheit 28 weist mehrere Kopfbandelemente 50, 50' auf, die jeweils dazu vorgesehen sind, sich zumindest teilweise um den Kopf 30 des Benutzers 20 zu erstrecken. Die Kopfbefestigungseinheit 28 weist drei Kopfbandelemente 50, 50' auf, die jeweils dazu vorgesehen sind, sich in verschiedenen Ebenen um den Kopf 30 des Benutzers 20 zu erstrecken. Die drei Kopfbandelemente 50, 50' sind bandförmig ausgebildet. Ein erstes Kopfbandelement 50 erstreckt sich von einem ersten gemeinsamen Verbindungspunkt 52 oberhalb eines Ohrs des Benutzers 20 um einen Hinterkopf des Kopfes 30 des Benutzers 20 zu einem zweiten gemeinsamen Verbindungspunkt oberhalb des anderen Ohrs des Benutzers 20. Eine effektive Länge des ersten Kopfbandelements 50 ist einstellbar. Die Kopfbefestigungseinheit 28 weist an dem Kopfbandelement 50 ein Einstellelement 54 auf. Das Einstellelement 54 ist von einem Einstellrad gebildet, welches beispielsweise von Helmen bekannt ist, mittels welchem durch Drehen eine effektive Länge des ersten Kopfbandelements 50 eingestellt werden kann. Ein zweites Kopfbandelement 50' erstreckt sich von dem ersten gemeinsamen Verbindungspunkt 52 über einen Oberkopf des Kopfes 30 des Benutzers 20 zu dem zweiten gemeinsamen Verbindungspunkt oberhalb des anderen Ohrs des Benutzers 20. Das zweite Kopfbandelement 50' erstreckt sich von dem ersten gemeinsamen Verbindungspunkt 52 annähernd in einem Winkel von 120° gegenüber dem ersten Kopfbandelement 50. Eine effektive Länge des zweiten Kopfbandelements 50' ist einstellbar. Das zweite Kopfbandelement 50' ist dazu zweiteilig ausgebildet. Die zwei Teile des zweiten Kopfbandelements 50' sind über eine verstellbare Verschlusseinheit verbunden. Die verstellbare Verschlusseinheit ist von einem Snapback-Verschluss gebildet. Ein drittes Kopfbandelement erstreckt sich von dem ersten gemeinsamen Verbindungspunkt 52 um eine Stirn des Kopfes 30 des Benutzers 20 zu dem zweiten gemeinsamen Verbindungspunkt oberhalb des anderen Ohrs des Benutzers 20. Das dritte Kopfbandelement erstreckt sich von dem ersten gemeinsamen Verbindungspunkt 52 annähernd in einem Winkel von 90° gegenüber dem zweiten Kopfbandelement 50'. Die drei Kopfbandelemente 50, 50' der Kopfbefestigungseinheit 28 sind über die Verbindungspunkte 52 fest miteinander verbunden. Die Kopfbefestigungseinheit 28 besteht aus einem teilweise elastischen Kunststoff. Grundsätzlich wäre jedoch auch ein anderes, einem Fachmann als sinnvoll erscheinendes Material denkbar.

Ferner weist die Laserschutzvorrichtung 10 eine Einstelleinheit 32 auf. Mittels der Einstelleinheit 32 ist eine Position der Schildeinheit 18 definiert relativ zu der Kopfbefestigungseinheit 28 beweglich ausgeführt. Mittels der Einstelleinheit 32 kann eine Position der Schildeinheit 18 definiert relativ zu der Kopfbefestigungseinheit 28 verstellt werden. Durch die Einstelleinheit 32 ist eine Position der Schildeinheit 18 in zumindest zwei Betriebsstellungen relativ zu der zumindest einen Kopfbefestigungseinheit 28 fixierbar ausgeführt. Mittels der Einstelleinheit 32 ist eine Position der Schildeinheit 18 in einem begrenzten Bereich in einer beliebigen Betriebsstellung relativ zu der zumindest einen Kopfbefestigungseinheit 28 fixierbar ausgeführt. Mittels der Einstelleinheit 32 kann eine Lage der Schildeinheit 18 in einer Betriebsstellung frei gewählt werden, wobei die Lage der Schildeinheit 18 fixiert werden kann. Die Schildeinheit 18 ist über die Einstelleinheit 32 mit der Kopfbefestigungseinheit 28 verbunden. Die Einstelleinheit 32 ist an den zwei Verbindungspunkten 52, 52' mit der Kopfbefestigungseinheit 28 verbunden. Ferner ist die Einstelleinheit 32 in einem Randbereich des Stirnbereichs 44 der Schildeinheit 18 mit der Schildeinheit 18 verbunden. Die Einstelleinheit 32 umfasst ein Grundelement. Das Grundelement ist an dem ersten Verbindungspunkt 52 der Kopfbefestigungseinheit 28 fest mit der Kopfbefestigungseinheit 28 verbunden. Ferner umfasst die Einstelleinheit 32 ein zu dem Grundelement spiegelsymmetrisches zweites Grundelement. Das zweite Grundelement ist an dem zweiten Verbindungspunkt der Kopfbefestigungseinheit 28 fest mit der Kopfbefestigungseinheit 28 verbunden.

Ferner weist die Einstelleinheit 32 ein Drehrad 56 auf, welches drehbar an der Schildeinheit 18 gelagert ist. Ferner ist das Drehrad 56 in einer Führungsschiene des ersten Grundelements translatorisch geführt. Das Drehrad 56 ist in der Führungsschiene des ersten Grundelements senkrecht zu der Kopfachse geführt. Mittels der Einstelleinheit 32 ist eine direkte Distanz zwischen den Augen 34 des Benutzers 20 und dem optischen Blendschutzfilter 12 einstellbar ausgebildet. Mittels der Einstelleinheit 32 kann eine direkte Distanz d₁, d₂ zwischen den Augen 34 des Benutzers 20 und dem optischen Blendschutzfilter 12 in horizontaler Richtung bzw. senkrecht zu der Kopfachse eingestellt werden. Die direkte Distanz d₁, d₂ zwischen den Augen 34 des Benutzers 20 und dem optischen Blendschutzfilter 12 kann mittels der Drehräder 56 der Einstelleinheit 32 verändert werden. Mittels der Drehräder 56 kann eine Position der Schildeinheit 18 in einer Längsrichtung relativ zu dem Gesicht des Benutzers 20 verändert werden. Ist eine gewünschte Längsposition gefunden, kann die Position durch Verdrehen der Drehräder 56 fixiert werden. Ferner ist mittels der Einstelleinheit 32 in einer Betriebsstellung ein Winkel der Schildeinheit 18 relativ zu der Kopfbefestigungseinheit 28 einstellbar ausgebildet. Die Einstelleinheit 32 weist dazu einen Hebel 58 auf.

Die Einstelleinheit 32 ist gemäß der EP 3 213 726 A1 ausgeführt. Eine Beschreibung der Einstelleinheit 32 der EP 3 213 726 A1 ist daher auch auf die vorliegende Beschreibung anwendbar und soll als Teil der Offenbarung angesehen werden.

Ferner weist die Laserschutzvorrichtung 10 eine Strahlenschutzeinheit 22 auf. Die Strahlenschutzeinheit 22 erstreckt sich um den Blendschutzfilter 12 und besteht zumindest teilweise aus einem laserabsorbierenden, insbesondere metallischen, Werkstoff. Die Strahlenschutzeinheit 22 weist eine sich um den Blendschutzfilter 12 erstreckende Strahlenschutzwandung 24 auf, welche aus einem metallischen Werkstoff besteht. Die Strahlenschutzwandung 24 besteht aus Aluminium und/oder einer Aluminiumlegierung. Die Strahlenschutzwandung 24 ist von einem geformten Blechteil gebildet, welches sich an einer Außenkante des optischen Blendschutzfilters 12 um den optischen Blendschutzfilter 12 erstreckt. Die Strahlenschutzwandung 24 erstreckt sich vollständig umlaufend entlang einer Außenkante des optischen Blendschutzfilters 12. Die Strahlenschutzwandung 24 liegt direkt an den Blendschutzfilter 12 an. Die Strahlenschutzwandung 24 liegt direkt an einem Rahmen des optischen Blendschutzfilters 12 an. Die Strahlenschutzwandung 24 ist von einem schmalen, umlaufenden Steg gebildet, welcher in einem getragenen Zustand dazu vorgesehen ist, sich in Richtung eines Gesichts des Benutzers 20 zu erstrecken. Die Strahlenschutzwandung 24 umfasst mehrere Teilbereiche. Die Strahlenschutzwandung 24 weist eine linke und rechte Seitenteilwandung 84, 84' auf, welche jeweils ein Sichtfeld des Benutzers 20 zu einer Seite hin abschirmen. Die Seitenteilwandungen 84, 84' weisen jeweils eine rechteckige Grundform auf. Die Seitenteilwandungen 84, 84' erstrecken sich insbesondere vertikal. Ferner weist die Strahlenschutzwandung 24 eine Stirnteilwandung 86 auf, welche ein Sichtfeld des Benutzers 20 nach oben hin abschirmt. Die Stirnteilwandung 86 erstreckt sich im Wesentlichen entlang einer Stirn des Benutzers 20. Die Stirnteilwandung 86 erstreckt sich insbesondere horizontal. Die Stirnteilwandung 86 grenzt im Wesentlichen senkrecht an die Seitenteilwandungen 84, 84` an. Die Stirnteilwandung 86 weist eine einseitig geschwungene Grundform auf. Des Weiteren weist die Strahlenschutzwandung 24 eine Nasenteilwandung 88 auf, welche ein Sichtfeld des Benutzers 20 nach unten hin abschirmt. Die Nasenteilwandung 88 erstreckt sich im Wesentlichen entlang der Wangen und der Nase des Benutzers 20. Die Nasenteilwandung 88 folgt dem Nasenausschnitt 14. Die Nasenteilwandung 88 erstreckt sich insbesondere horizontal. Die Nasenteilwandung 88 grenzt im Wesentlichen senkrecht an die Seitenteilwandungen 84, 84` an. Die Nasenteilwandung 88 weist eine einseitig geschwungene Grundform auf. Eine Höhe des Steges variiert dabei derart, dass die Strahlenschutzwandung 24 einer Kontur des Gesichts zumindest teilweise folgt. Die Strahlenschutzwandung 24 weist links und rechts an den Seitenteilwandungen 84, 84` insbesondere eine maximale Höhe auf, während die Höhe in einem Bereich der Stirnteilwandung 86 zu einer Mitte hin kontinuierlich verringert ist. Ferner ist die Höhe der Strahlenschutzwandung 24 in einem Bereich der Nasenteilwandung 88 minimal, wobei die Höhe der Strahlenschutzwandung 24 kontinuierlich zu einer Mitte des Nasenausschnitts 14 abnimmt. Die Strahlenschutzwandung 24 ragt zumindest im Wesentlichen senkrecht zu einer Haupterstreckungsebene des Blendschutzfilters 12 in einen Innenraum der Schildeinheit 18. Die Höhe der Strahlenschutzwandung 24 bezieht sich daher insbesondere auf eine Richtung senkrecht zu der Haupterstreckungsebene des Blendschutzfilters 12. Die Strahlenschutzwandung 24 ragt in einem getragenen Zustand zumindest im Wesentlichen senkrecht zu einer Haupterstreckungsebene des Blendschutzfilters 12 in einen Innenraum der Schildeinheit 18 in Richtung eines Gesichts des Benutzers 20. Die Strahlenschutzwandung 24 ist beispielhaft mit der Schutzkassette 38 verschraubt. Die Strahlenschutzwandung 24 weist dazu auf beiden Seiten Befestigungslaschen 80, 80' auf, durch welche Schrauben 82, 82' zu einer Verbindung mit der Schutzkassette 38 geschraubt werden können. Es sind jedoch auch andere, einem Fachmann als sinnvoll erscheinende Verbindungstechniken denkbar.

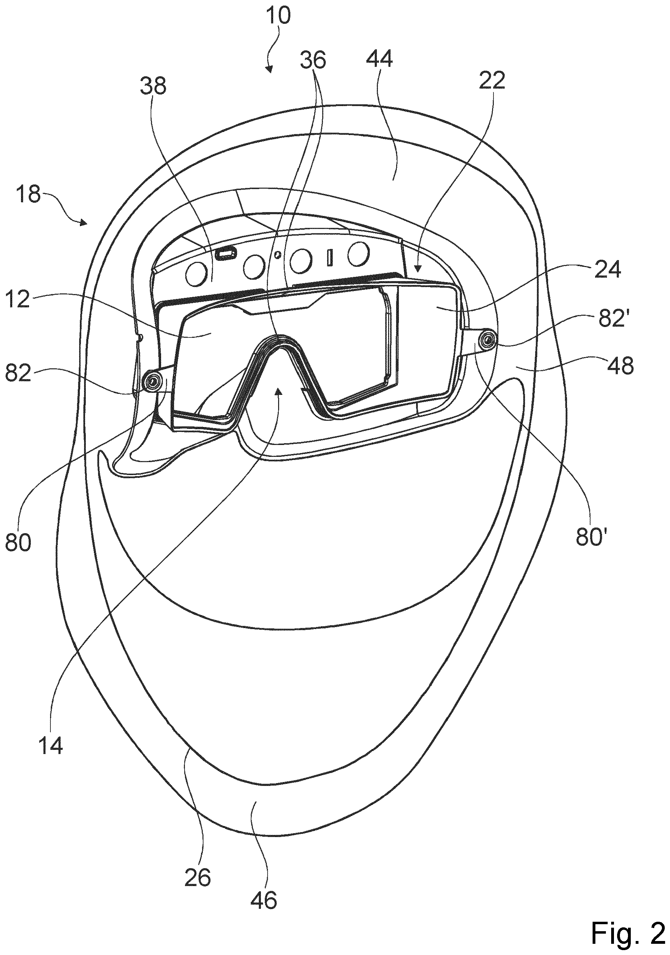

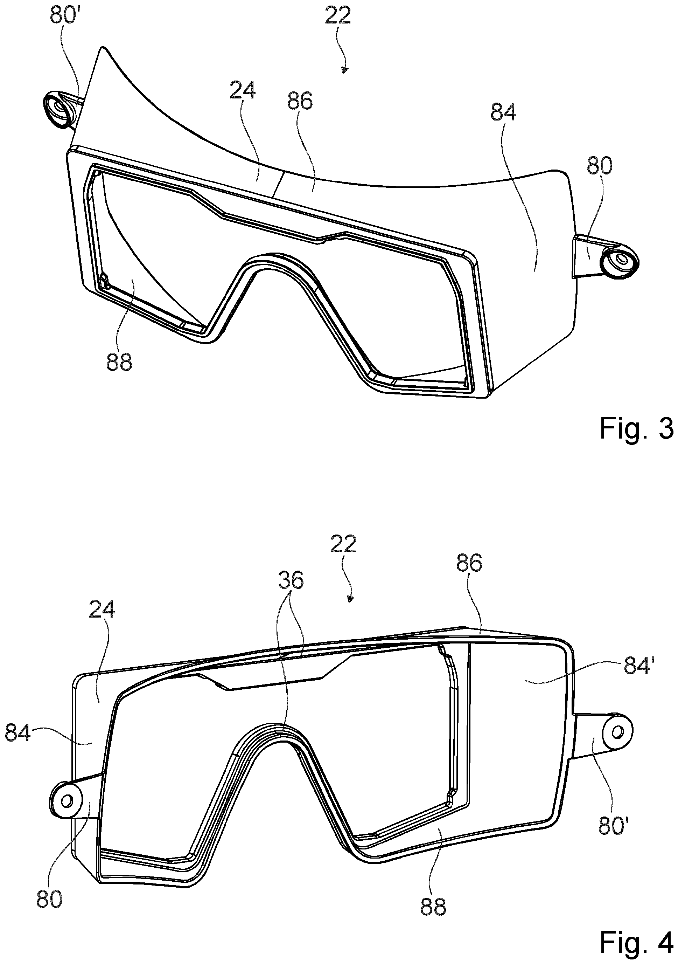

Des Weiteren weist die Strahlenschutzeinheit 22 einen Schildeinsatz 26 auf, welcher aus einem laserabsorbierenden, insbesondere metallischen, Werkstoff besteht. Der Schildeinsatz 26 besteht zumindest zu einem Großteil aus Aluminium und/oder einer Aluminiumlegierung. Der Schildeinsatz 26 ist direkt mit der Schildeinheit 18 gekoppelt und verstärkt diese. Der Schildeinsatz 26 bildet eine bereichsweise Zweiwandigkeit der Schildeinheit 18 aus. Der Schildeinsatz 26 liegt an einer Innenseite der Schildeinheit 18 an der Schildeinheit 18 an, wobei der Schildeinsatz 26 einer Form der Schildeinheit 18 folgt. Der Schildeinsatz 26 ist in einem Mundbereich eines Benutzers 20 hinter der Schildeinheit 18 angeordnet. Der Schildeinsatz 26 ist in dem Kinnbereich 46 der Schildeinheit 18 angeordnet. Der Schildeinsatz 26 erstreckt sich über einen Großteil des Kinnbereichs 46 der Schildeinheit 18. Der Schildeinsatz 26 erstreckt sich zumindest teilweise in den Nasenausschnitt 14 hinein. Der Schildeinsatz 26 ist mit der Schildeinheit 18 verklebt. Es sind jedoch auch andere, einem Fachmann als sinnvoll erscheinende Verbindungstechniken denkbar (Figur 2, 3, 4).

Die Strahlenschutzeinheit 22 ist von der Schildeinheit 18 verschieden. Die Strahlenschutzeinheit 22 ist fest mit der Schildeinheit 18 verbunden. Die Strahlenschutzeinheit 22 ist über die Schutzkassette 38 fest mit der Schildeinheit 18 verbunden.

Zudem weist die Strahlenschutzeinheit 22 einen Gesichtsanschlag 36 auf. Der Gesichtsanschlag 36 ist in einer Endposition der Strahlenschutzeinheit 22 dazu vorgesehen ein Gesicht des Benutzers 20 zu kontaktieren. Eine Anschlagsposition ist dabei insbesondere durch eine Berührung zwischen Gesicht und Gesichtsanschlag 36 definiert. Eine Anschlagsposition definiert dabei eine Betriebsstellung. Der Gesichtsanschlag 36 der Strahlenschutzeinheit 22 ist zu einer Positionierung der Strahlenschutzeinheit 22 relativ zu einem Gesicht eines Benutzers 20 vorgesehen. Der Gesichtsanschlag 36 ist von einer Nasen- und/oder Stirnauflage gebildet. Der Gesichtsanschlag 36 ist beispielhaft von einer brillenähnlichen Nasenauflage gebildet. Der Gesichtsanschlag 36 ist in einer Betriebsstellung zu einer Kontaktierung einer Nase und/oder einer Stirn des Benutzers 20 vorgesehen. Der Gesichtsanschlag 36 ist in einer Betriebsstellung zu einer Kontaktierung eines Nasenrückens der Nase des Benutzers 20 vorgesehen. Hierdurch kann zuverlässig eine Endposition der Strahlenschutzeinheit 22 definiert werden. Insbesondere kann dadurch eine zuverlässige Positionierung der Strahlenschutzeinheit 22 relativ zu den Augen 34 des Benutzers 20 erreicht werden. Der Gesichtsanschlag 36 ist in einem Bereich des Nasenausschnitts 14 des optischen Blendschutzfilters 12 angeordnet. Grundsätzlich wäre jedoch auch eine andere, einem Fachmann als sinnvoll erscheinende Ausgestaltung und/oder Anordnung des Gesichtsanschlags 36 denkbar (Figur 2).

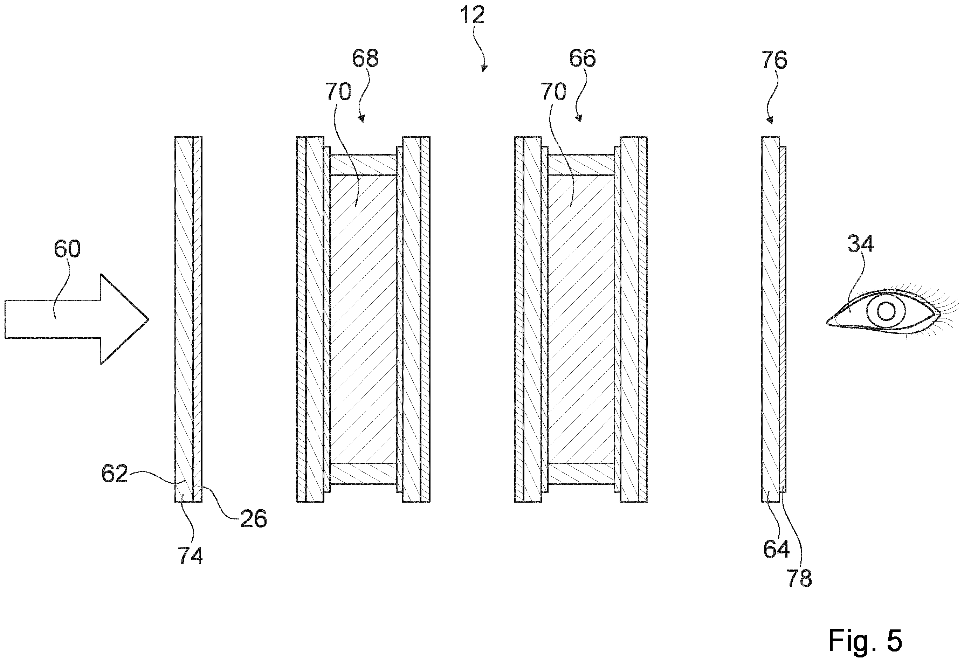

Fig. 5 zeigt eine schematische Seitenansicht des optischen Blendschutzfilters 12. Eine typische Einfallrichtung 60 für abzuschirmende elektromagnetische Strahlung ist in Pfeilrichtung angezeigt. Eine Position eines Benutzers ist auf einer der Einfallrichtung 60 gegenüberliegenden Seite des optischen Blendschutzfilters 12 durch ein Auge 34 angedeutet. Der optische Blendschutzfilter 12 weist eine Abdeckscheibe 62 auf. Der optische Blendschutzfilter 12 weist eine weitere Abdeckscheibe 64 auf. Ferner weist der optische Blendschutzfilter 12 eine Flüssigkristallzelle 66 und eine weitere Flüssigkristallzelle 68 auf. Die Flüssigkristallzelle 66 und die weitere Flüssigkristallzelle 68 sind in Sichtrichtung zwischen der Abdeckscheibe 62 und der weiteren Abdeckscheibe 64 angeordnet. Die Abdeckscheibe 62 ist auf einer dem Benutzer 20 im getragenen Zustand abgewandten Seite des optischen Blendschutzfilters 12 angeordnet. Die weitere Abdeckscheibe 64 ist auf einer dem Benutzer 20 im getragenen Zustand zugewandten Seite des optischen Blendschutzfilters 12 angeordnet. Die Abdeckscheibe 62 und die weitere Abdeckscheibe 64 sind zu einem Schutz der Flüssigkristallzelle 66 und der weiteren Flüssigkristallzelle 68 und deren Bestandteile vorgesehen. Die Flüssigkristallzellen 66, 68 weisen jeweils eine aktive Schicht 70 auf. Die aktive Schicht 70 ist dazu vorgesehen, eine Polarisationsrichtung einfallenden Lichts mittels einer Ansteuerung durch die Elektronikeinheit 40 zu manipulieren. Die aktive Schicht 70 ist dazu vorgesehen, abhängig von einer anliegenden Spannung die Polarisationsrichtung von Lichtstrahlen zu drehen. Die aktive Schicht 70 ist zumindest teilweise flüssig. Die aktive Schicht 70 ist als eine Flüssigkristallschicht ausgebildet.

Der optische Blendschutzfilter 12 weist eine passive Filtereinheit 72 auf. Die passive Filtereinheit 72 weist ein absorptives Infrarotfilterelement 74 auf. Das Infrarotfilterelement 74 ist als ein Absorptionsglas ausgebildet. Das Infrarotfilterelement 74 ist einstückig mit der Abdeckscheibe 62 ausgebildet.

Der optische Blendschutzfilter 12 weist eine Antireflexionseinheit 76 auf. Die Antireflexionseinheit 76 weist eine Antireflexbeschichtung 78 auf. Die Antireflexbeschichtung 78 ist auf einer Oberfläche der weiteren Abdeckscheibe 64 angeordnet. Die Antireflexbeschichtung 78 ist auf der im getragenen Zustand einem Benutzer 20 zugewandten Oberfläche der Abdeckscheibe 64 angeordnet. Die Antireflexbeschichtung 78 ist als ein Interferenzfilter ausgebildet. Grundsätzlich wären jedoch auch andere, einem Fachmann als sinnvoll erscheinende Ausbildungen der Antireflexbeschichtung 78 denkbar. Auf der weiteren Abdeckscheibe 64 kann ferner ein Farbfilter vorgesehen sein.

Die weitere Abdeckscheibe 64 ist insbesondere von einer tauschbaren Innenschutzscheibe gebildet, welche für verschiedene Wellenlängen getauscht werden kann.

### Bezugszeichenliste

- 10: Laserschutzvorrichtung
- 12: Blendschutzfilter
- 14: Nasenausschnitt
- 16: Sensoreinheit
- 18: Schildeinheit
- 20: Benutzer
- 22: Strahlenschutzeinheit
- 24: Strahlenschutzwandung
- 26: Schildeinsatz
- 28: Kopfbefestigungseinheit
- 30: Kopf
- 32: Einstelleinheit
- 34: Auge
- 36: Gesichtsanschlag
- 38: Schutzkassette
- 40: Elektronikeinheit
- 42: Vorsatzscheibe
- 44: Stirnbereich
- 46: Kinnbereich
- 48: Augenbereich
- 50: Kopfbandelement
- 52: Verbindungspunkt
- 54: Einstellelement
- 56: Drehrad
- 58: Hebel
- 60: Einfallrichtung
- 62: Abdeckscheibe
- 64: Abdeckscheibe
- 66: Flüssigkristallzelle
- 68: Flüssigkristallzelle
- 70: Schicht
- 72: Filtereinheit
- 74: Infrarotfilterelement
- 76: Antireflexionseinheit
- 78: Antireflexbeschichtung
- 80: Befestigungslasche
- 82: Schraube
- 84: Seitenteilwandung
- 86: Stirnteilwandung
- 88: Nasenteilwandung

## Patentansprüche

1. Laserschutzvorrichtung, insbesondere Laserschutzhelm, mit zumindest einem optischen Blendschutzfilter (12), welcher insbesondere einen Nasenausschnitt (14) aufweist, mit zumindest einer Sensoreinheit (16), welche zu einer Erfassung eines Arbeitszustands und einer davon abhängigen Ansteuerung einer Durchlässigkeit des Blendschutzfilters (12) vorgesehen ist, mit zumindest einer Schildeinheit (18), welche dazu vorgesehen ist, ein Gesicht eines Benutzers (20) zu schützen und in welcher der zumindest eine Blendschutzfilter (12) fest aufgenommen ist,
**gekennzeichnet durch**
zumindest eine Strahlenschutzeinheit (22), welche sich um den Blendschutzfilter (12) erstreckt und zumindest teilweise aus einem laserabsorbierenden, insbesondere metallischen, Werkstoff besteht.

2. Laserschutzvorrichtung nach Anspruch 1,
**dadurch gekennzeichnet, dass**
die zumindest eine Strahlenschutzeinheit (22) zumindest eine sich um den Blendschutzfilter (12) erstreckende Strahlenschutzwandung (24) aufweist, welche aus einem laserabsorbierenden, insbesondere metallischen, Werkstoff besteht.

3. Laserschutzvorrichtung nach Anspruch 2,
**dadurch gekennzeichnet, dass**
die zumindest eine Strahlenschutzwandung (24) aus Aluminium und/oder einer Aluminiumlegierung besteht.

4. Laserschutzvorrichtung zumindest nach Anspruch 2,
**dadurch gekennzeichnet, dass**
die zumindest eine Strahlenschutzwandung (24) zumindest im Wesentlichen senkrecht zu einer Haupterstreckungsebene des Blendschutzfilters (12) in einen Innenraum der Schildeinheit (18) ragt.

5. Laserschutzvorrichtung nach einem der vorhergehenden Ansprüche,
**dadurch gekennzeichnet, dass**
die zumindest eine Strahlenschutzeinheit (22) zumindest einen Schildeinsatz (26) aufweist, welcher aus einem laserabsorbierenden, insbesondere metallischen, Werkstoff besteht.

6. Laserschutzvorrichtung nach einem der vorhergehenden Ansprüche,
**dadurch gekennzeichnet, dass**
der Schildeinsatz (26) in einem Mundbereich eines Benutzers hinter der Schildeinheit (18) angeordnet ist.

7. Laserschutzvorrichtung nach einem der vorhergehenden Ansprüche,
**dadurch gekennzeichnet, dass**
die Strahlenschutzeinheit (22) von der Schildeinheit (18) verschieden ist.

8. Laserschutzvorrichtung nach einem der vorhergehenden Ansprüche,
**gekennzeichnet durch**
eine Kopfbefestigungseinheit (28) zu einer Befestigung an einem Kopf (30) eines Benutzers (20) und eine Einstelleinheit (32), durch welche eine Position der zumindest einen Schildeinheit (18) definiert relativ zu der zumindest einen Kopfbefestigungseinheit (28) beweglich ausgeführt ist.

9. Laserschutzvorrichtung nach einem der vorhergehenden Ansprüche,
**dadurch gekennzeichnet, dass**
mittels der zumindest einen Einstelleinheit (32) eine direkte Distanz zwischen den Augen (34) des Benutzers und dem optischen Blendschutzfilter (12) einstellbar ausgebildet ist.

10. Laserschutzvorrichtung nach einem der vorhergehenden Ansprüche,
**dadurch gekennzeichnet, dass**
die zumindest eine Strahlenschutzeinheit (22) zumindest einen Gesichtsanschlag (36) aufweist, welcher in einer Endposition der Strahlenschutzeinheit (22) dazu vorgesehen ist, ein Gesicht des Benutzers (20) zu kontaktieren.

11. Strahlenschutzeinheit einer Laserschutzvorrichtung (10) nach einem der vorhergehenden Ansprüche.
